# EUROPEAN PATENT SPECIFICATION

(11) **EP 2 651 251 B1**
(45) Date of publication and mention of the grant of the patent: **01.06.2016**
(21) Application number: 11813358.6
(22) Date of filing: 19.12.2011
(51) Int. Cl.: A61K 31/385, A61K 31/519, A61P 1/16, A61P 1/18, A61P 3/04, A61P 3/06, A61P 3/08, A61P 5/24, A61P 9/10, A61K 31/6615, A23L 33/15

(54) **COMPOSITION FOR THE TREATMENT OF INFERTILITY**
ZUSAMMENSETZUNG ZUR BEHANDLUNG VON UNFRUCHTBARKEIT
COMPOSITION POUR LE TRAITEMENT DE L'INFERTILITÉ

(30) Priority: 17.12.2010 IT MI20102313
(43) Date of publication of application: 23.10.2013
(73) Proprietor: Laborest Italia S.R.L., 20014 Nerviano (IT)
(72) Inventor: AMELOTTI, Luigi, I-20014 Nerviano (MI) (IT); SECONDINI, Lorenzo, I-20014 Nerviano (MI) (IT)
(74) Representative: Hoffmann Eitle
(86) International application number: PCT/IB2011/003085
(87) International publication number: WO 2012/080831

(56) References cited:
- US-A1- 2002 155 163
- US-A1- 2007 065 456
- US-A1- 2007 160 590
- PAPALEO E ET AL: "Myo-inositol in patients with polycystic ovary syndrome: a novel method for ovulation inducti", GYNECOLOGICAL ENDOCRINOLOGY, vol. 23, no. 12, 1 December 2007 (2007-12-01), pages 700-703, XP008098721, PARTHENON PUBLISHING, LONDON, GB ISSN: 0951-3590, DOI: 10.1080/09513590701672405
- Chris D. Meletis, Nieske Zabriskie: "Natural Approaches for Treating Polycystic Ovary Syndrome", Alternative and complementary therapies , vol. 12, no. 4 7 August 2006 (2006-08-07), pages 157-164, XP002635366, Retrieved from the Internet: URL:http://www.liebertonline.com/doi/pdf/1 0.1089/act.2006.12.157 [retrieved on 2011-05-05]
- ANONYMOUS: "CapsuDar and AsparCote - Microencapsulated Ingredients Product Line", LycoRed , 2005, XP002635367, Retrieved from the Internet: URL:http://www.lycored.com/web/content/mic roencaps-Ingredients07.asp [retrieved on 2011-05-06]
- United States Department of Agriculture: "Nutrient data for 09297, Raisins, golden seedless", National Nutrient Database for Standard Reference , vol. Release 24 30 March 2012 (2012-03-30), pages 1-1, XP002673631, Retrieved from the Internet: URL:http://ndb.nal.usda.gov/ndb/foods/show /2471 [retrieved on 2012-04-10]
- Umesh Masharani ET AL: "Effects of Controlled-Release Alpha Lipoic Acid In Lean, Nondiabetic Patients with Polycystic Ovary Syndrome Author Affiliations: Corresponding Author", Journal of Diabetes Science and Technology Volume Diabetes Technology Society, 1 March 2010 (2010-03-01), pages 359-364, XP055115956, Retrieved from the Internet: URL:http://www.ncbi.nlm.nih.gov/pmc/articl es/PMC2864173/pdf/dst-04-0359.pdf [retrieved on 2014-04-30]

## Description

The present invention relates to a pharmaceutical composition or a nutraceutical composition or a supplement product for use in the treatment of infertility.

Polycystic Ovary Syndrome (PCOS) is an endocrine disorder which affects 10-15% of the female population. This syndrome represents the most common hormonal disorder in women of reproductive age. Above all, it is the main cause of oligo-amenorrhoea and female infertility. The symptoms and severity of the disease vary greatly among women. Polycystic ovary syndrome has a basic etiology linked to an oxidative syndrome with insulin resistance. The insulin resistance and consequent hyperinsulinaemia that are often associated with this syndrome play an important role not only in the pathogenesis of the syndrome itself, but also in the clinical symptomatology.

Moreover, patients with this syndrome have an increased risk of developing pathologies such as type 2 diabetes, the metabolic syndrome in connection with hypertension, dislipidaemia and cardiovascular diseases, obesity, high plasma levels of C-Reactive Protein (CRP) and triglycerides with low levels of cholesterol.

The use of folic acid, inositol and/or lipoic acid in nutritional supplements and/or pharmaceutical compositions has been reported.

For instance, US 2002/0155163 A1 describes a daily multi-vitamin and mineral combination for use in the adjunct care of humans with asthma or diabetes. The combination comprises first, second and third formulations which together provide *inter alia* folate, inositol and alpha-lipoic acid.

US 2007/0160590 A1 describes a low carbohydrate/high protein bar for comprising inter alia R-alpha-lipoic acid, myo-inositol, and raisin puree. Raisins are reported to be a source of various vitamins, including folate.

US 2007/0065456 A1 describes nutritional supplement formulations comprising inter alia inositol, lipoic acid and folic acid which are reported to enhance athletic performance, reduce weight, detoxify the blood, liver and/or GI tract, as well as reduce inflammation.

In *"*Alternative and Complementary Therapies" (2006, v. 12(4), pp. 157-164), Meletis and Zabriskie outline various conventional (e.g. Matformin) and nutrient/herbal treatments (e.g. alpha-lipoic acid, D-chiro-inositol) that have been used or are suggested for the treatment of various PCOS-related symptoms.

In *"*Gynecological Endocrinology" (2007, v. 23(12), pp. 700-703), Papaleo et al. reported that, of 25 PCOS women suffering from ovulation dysfunction and who were treated twice daily with an orally administered composition comprising 2 g myo-inositol and 200 µg folic acid, 10 biochemical pregnancies occurred.

In the "Journal of Diabetes Science and Technology" (2010, v. 4(2), pp. 359-364), Masharani et al. reported that controlled-release alpha lipoic acid significantly increased insulin sensitivity and that for two subjects in the study, an increased number of menstrual cycles was observed.

The use of folic acid, on its own and as an adjuvant in association with the active ingredient metformin, for treating polycystic ovary syndrome is well known. However, satisfactory results are not always achieved with such treatments.

Thus there remains a need to have new and effective formulations capable of treating and/or reducing the metabolic disorders deriving from or connected to a polycystic ovary syndrome, non-alcoholic hepatic steatosis, cirrhosis, hyperlipidaemia, atherosclerosis, infertility and alterations of the menstrual cycle.

In particular, there remains a need to have new and effective formulations that do not have any side effects, are well tolerated by the subjects treated and are easy to administer.

The present invention relates to a pharmaceutical composition or a nutraceutical composition or a supplement product for use in the treatment of infertility.

In particular, the invention relates to:
(i) A composition comprising:
   - vitamin B9 or folic acid or folacin, and
   - myo-inositol, and
   - alpha lipoic acid,
   for use in the treatment of infertility.
(ii) The composition for use in accordance with (i) above, wherein said alpha lipoic acid is present as a 50/50 racemic R-(+)-alpha lipoic acid/S-(-)-alpha lipoic acid mixture.
(iii) The composition for use in accordance with (i) or (ii) above, wherein said acid alpha lipoic is present in microencapsulated or coated form.
(iv) The composition for use in accordance with any one of (i) to (iii) above, wherein said composition consists of:
   - vitamin B9 or folic acid or folacin, preferably pteroylmonoglutamic acid;
   - myo-inositol;
   - alpha lipoic acid in the form of a 50/50 racemic R-(+)-alpha lipoic acid/S-(-)-alpha lipoic acid mixture, and excipients and technological additives.
(v) The composition for use in accordance with any one of (i) to (iv) above, wherein said composition is in solid form for oral administration, preferably tablets or granules for sachets or hard capsules.
(vi) The composition for use in accordance with any one of (i) to (v) above, as a pharmaceutical composition or supplement product.

Other preferred embodiments of the present invention are set forth in the detailed description that follows.

In the context of the present invention, "formulation" means a pharmaceutical composition or a nutraceutical composition or a supplement product. The formulation comprises an association of compounds (i), (ii) and (iii), as described below, and excipients, technological additives, co-formulations, polar and/or semipolar polymeric matrices, carriers and stabilizers of pharmaceutical grade or acceptable for the body.

For background information purposes, the Applicant has found that a formulation comprising or, alternatively, consisting of an association among:
- at least one vitamin selected from the group comprising or, alternatively, consisting of B group vitamins,
- an inositol compound or a derivative thereof, and
- a lipoic acid or a derivative thereof,
is capable of being used in:
(a) a preventive and/or curative treatment of the metabolic disorders deriving from or connected to a polycystic ovary syndrome, non-alcoholic hepatic steatosis, cirrhosis, hyperlipidaemia, atherosclerosis, infertility and alterations of the menstrual cycle - treatment (a);
(b) a curative treatment for reducing and/or eliminating the metabolic disorders deriving from or connected to a polycystic ovary syndrome, non-alcoholic hepatic steatosis, cirrhosis, hyperlipidaemia, atherosclerosis, infertility and alterations of the menstrual cycle - treatment (b);
(c) a treatment for reducing and/or eliminating the pain or symptoms connected with the perception of pain in polycystic ovary syndrome, and alterations of the menstrual cycle - treatment (c).

In many cases, female infertility occurs as a result of chronic anovulation. The alterations of the menstrual cycle comprise, for example, oligomenorrhoea and amenorrhoea.

The formulation of the present invention comprises or, alternatively, consists of:
(i) vitamin B9 or folic acid or folacin, in a therapeutically effective amount,
(ii) myo-inositol, in a therapeutically effective amount, and
(iii) alpha-lipoic acid, in a therapeutically effective amount, for use in the treatment of infertility.

The formulation also comprises, in addition to the association of compounds (i), (ii) and (iii), excipients, technological additives, co-formulants, polar and/or semipolar polymeric matrices, carriers and stabilizers, all of the above pharmaceutically acceptable.

For example, the pharmacologically acceptable excipients are selected from among thickening agents, such as, for example, xanthan gum and guar gum; sweeteners, such as, for example, sorbitol and sucralose; acidifiers, such as, for example citric acid; maltodextrins; anti-agglomerants, such as, for example silicon dioxide and magnesium stearate; antioxidants and flavourings.

The vitamin B present in formulation -(i) is selected from among vitamin B9 or folic acid or folacin. The vitamin B can be present in the form of a pharmaceutically acceptable salt. The commercially available chemical form in which folic acid is sold is pteroylmonoglutamic acid. Advantageously, the formulation of the present invention contains folic acid.

The inositol compound present in formulation -(ii) is myo-inositol. Other forms of inositol include chiro-inositol for example, D-chiro-inositol (also known as 1D-chiro-inositol or D-(+)-chiro-inositol). Examples of inositol derivatives and analogues are, for example, pinitol, e.g. D-pinitol, or D-chiro-inositol-phosphate. The formulation of the present invention contains inositol in the form of myo-inositol (cis-1,2,3,5-trans-4,6-cyclohexanehexol). Myo-inositol is classified as a component of the B complex (being referred to as B8). Inositol is a polyalcohol classified as an insulin sensitizing agent. Myo-inositol exerts its insulin mediator function as inositol phosphoglycan (IPG). Myo-inositol is the most abundant form of inositol in nature, whereas D-chiro-inositol is synthesized by an epimerase which converts myo-inositol into D-chiro-inositol; this reaction is insulin dependent.

The lipoic acid present in formulation -(iii) contains alpha lipoic acid (thioctic acid) in the R(+) configuration (R (+) enantiomer) or as a racemic R(+)/S(-) mixture. The racemic mixture can contain the R(+) enantiomer in an amount comprised from 50% to 100%, relative to the lipoic acid present, and the S(-) enantiomer in an amount comprised from 50% to 0%, relative to the lipoic acid present.

The alpha lipoic acid can be present in the formulation only in the form of the R(+) enantiomer, or as a 50/50, or 60/40, 70/30, 80/20 or 90/10 racemic R-(+)-alpha lipoic acid/S-(-)-alpha lipoic acid mixture. Preferably, the formulation of the present invention contains a 50/50 racemic R-(+)-alpha lipoic acid/S-(-)-alpha lipoic acid mixture.

Advantageously, the formulation of the present invention contains alpha lipoic acid in non-dissociated acid form or in the form of a pharmaceutically acceptable salt, for example a sodium salt.

The amount of B group vitamin, inositol compound (or derivatives thereof) and lipoic acid (or derivatives thereof) in the formulation is preferably from 50 to 80% by weight, relative to the total weight of the formulation, preferably from 60 to 70% by weight, relative to the total weight of the formulation.

The amount of excipients (or other substances such as technological additives, co-formulants, polar and/or semipolar polymeric matrices, carriers and stabilizers) in the formulation is preferably from 50 to 20% by weight, relative to the total weight of the formulation, preferably from 40 to 20% by weight, relative to the total weight of the formulation.

The amount of B group vitamin present in the formulation is preferably from 0.001 to 0.10% by weight, relative to the total weight of the formulation, preferably from 0.005 to 0.010% by weight, relative to the total weight of the formulation. The formulation preferably comprises folic acid or vitamin B9 or folacin in an amount comprised from 0.001 to 0.10% by weight, relative to the total weight of the formulation, preferably from 0.005 to 0.010% by weight, relative to the total weight of the formulation.

The amount of inositol compound present in the formulation is preferably from 30 to 60% by weight, relative to the total weight of the formulation, preferably from 40 to 50% by weight, relative to the total weight of the formulation. The formulation preferably comprises myo-inositol in an amount comprised from 30 to 60% by weight, relative to the total weight of the formulation, preferably from 40 to 50% by weight, relative to the total weight of the formulation.

The amount of lipoic acid present in the formulation is preferably from 10 to 35% by weight, relative to the total weight of the formulation, preferably from 15 to 20% by weight, relative to the total weight of the formulation. The formulation preferably comprises alpha lipoic acid in the R(+) configuration (R(+) enantiomer) or as a racemic R(+)/S(-) mixture [where the racemic mixture contains the R(+) enantiomer in an amount comprised from 50% to 100%, relative to the lipoic acid present] in an amount comprised from 5 to 35% by weight, relative to the total weight of the formulation, preferably from 15 to 20% by weight, relative to the total weight of the formulation.

The folic acid is administered, via the formulation of the present invention, at a concentration comprised from 100 to 600 micrograms/daily dose, preferably from 200 to 400 micrograms/daily dose.

The myo-inositol is administered, via the formulation of the present invention, at a concentration comprised from 1 to 5 grams/daily dose, preferably from 2 to 4 grams/daily dose.

The formulation of the present invention contains alpha lipoic acid as the R(+) enantiomer, or as a 50/50 or 60/40, 70/30, 80/20 or 90/10 racemic mixture of R-(+)-alpha lipoic acid and S-(-)-alpha lipoic acid. Preferably, it is a 50/50 racemic mixture.

The alpha lipoic acid is protected to avoid pyrosis in the mouth, esophagus, and stomach (gastric pyrosis) of the subject undergoing treatment. Furthermore, the alpha lipoic acid is protected so as to release the lipoic acid in a gradual and controlled manner in the body of the subject treated with the formulation of the present invention.

It is desirable that at least 50%, preferably 60%, of the alpha lipoic acid be released in the stomach within 30 minutes after administration and the remaining 50%, preferably 40%, be released in the gastrointestinal tract. This release mode ensures a level of alpha lipoic acid in blood serum for a period comprised from 0 to 4 hours.

The gradual release of lipoic acid makes it possible, on the one hand, to obtain levels of lipoic acid in blood serum that are comparable to those that would be obtained with a single injectable dose or with a formulation containing non-controlled release (non-protected) lipoic acid and, on the other hand, to maintain, over a period of time comprised from 0 to 4 hours, a level of lipoic acid in blood serum comprised from 20 to 80 ng/ml of plasma, preferably from 30 to 60 ng/ml of plasma, even more preferably from 45 to 55 ng/ml of plasma.

The alpha lipoic acid present in formulation -(iii) can be in microencapsulated or coated form (protected form) so as to ensure a gradual and controlled release of lipoic acid in a time interval comprised from 0 to 4 hours.

Alternatively, the alpha lipoic acid is present in said formulation -(iii) not in a microencapsulated or coated form, but rather immersed in a matrix, for example of a polymeric nature, which in any case ensures a gradual and controlled release of the lipoic acid over time, in a time interval comprised from 0 to 4 hours (protected form). The choice will depend on the type of pharmaceutical administration it is desired to achieve with the formulation of the present invention.

The formulation of the present invention (pharmaceutical composition or nutraceutical composition or supplement product or food supplement) is prepared for oral administration, for example in the form of a tablet, granules for tablets or granules for sachets.

If the formulation of the present invention is in the form of a tablet, the alpha lipoic acid that is present in said formulation is mixed together with polymers, for example polymers selected from among microcrystalline cellulose, hydroxypropyl cellulose and hydroxypropyl methyl cellulose at a different molecular weight, and together with additives such as magnesium stearate, silicon dioxide, talc and dicalcium phosphate so as to achieve a controlled and gradual release. The coating is made using apparatus and technologies known to a person skilled in the art, who is undoubtedly capable of selecting the most suitable polymers among those commercially available and the best operating conditions.

Subsequently, once the tablet has been prepared, it is externally coated with a thin layer of a coating or film (film coating) to provide a coated tablet that ensures a controlled and gradual release in a period of from 0 to 4 hours. The external coating of the tablet is realized using apparatus and technologies known to a person skilled in the art, who is undoubtedly capable of selecting the most coating materials or films among those commercially available and the best operating conditions.

For example, a tablet comprising the formulation of the present invention is coated (protected tablet) with a thin layer of a coating or film (film coating) using one or more enteric coatings. The coating or film has a pH-dependent solubility capable of enabling release according to the pH value in a given segment of the gastrointestinal tract, and of ensuring a rapid absorption of the lipoic acid. The enteric coating is realized with coating compounds known to persons skilled in the art, for example hydroxypropyl cellulose, hydroxypropyl methylcellulose phthalate or polyvinyl acetate-phthalate. The coating is applied using apparatus and technologies known to a person skilled in the art, who is undoubtedly capable of selecting the most suitable coating compounds among those commercially available.

In the case of a tablet, it is desirable that at least 50%, preferably 60%, of alpha lipoic acid be released in the stomach within 30 minutes after administration and the remaining 50%, preferably 40%, be released in the gastrointestinal tract in the following 3-4 hours. This release mode ensures a level of alpha lipoic acid in blood serum for a period comprised from 0 to 4 hours. The tablet of the present invention can be said to have a 50/50 fast/slow controlled release.

If the formulation of the present invention is in the form of granules for sachets or for hard gelatine capsules, the alpha lipoic acid is present in said formulation in microencapsulated or coated form (encapsulated or coated lipoic acid) to provide a controlled and gradual release in a period of from 0 to 4 hours. In this case, the lipoic acid is microencapsulated or coated (protected form) to a degree of at least 92%, preferably 96%, by weight. The microencapsulation or coating is applied on the lipoic acid using apparatus and technologies known to a person skilled in the art, who is undoubtedly capable of selecting the most suitable coating compounds among those commercially available. In one embodiment, the alpha lipoic acid is microencapsulated or coated with a combination of lacquer gum, triethylcitrate and talc.

In the event of granules for sachets, it is desirable that at least 50%, preferably 60%, of alpha lipoic acid be released in the stomach within 30 minutes after administration and the remaining 50%, preferably 40%, be released in the gastrointestinal tract in the following 3-4 hours. This release mode ensures a level of alpha lipoic acid in blood serum for a period comprised from 0 to 4 hours. The granules for sachets of the present invention can be said to have a 50/50 fast/slow controlled release.

The amount of lipoic acid is administered, via the formulation of the present invention, at a concentration comprised from 100 to 1200 milligrams/daily dose, preferably from 300 to 800 milligrams/daily dose. The lipoic acid or alpha lipoic acid is administered, via the formulation of the present invention, at a concentration comprised from 100 to 1200 milligrams/daily dose, preferably from 300 to 600 milligrams/daily dose, as the R(+) enantiomer or as a 50/50 or 60/40, 70/30, 80/20or 90/10 racemic mixture of R-(+)-alpha lipoic acid and S-(-)-alpha lipoic acid. Preferably, as a 50/50 racemic mixture.

Advantageously, the formulation of the present invention contains R-(+)-alpha lipoic acid or a 50/50 or 60/40, 70/30, 80/20 or 90/10 racemic mixture of R-(+)-alpha lipoic acid and S-(-)-alpha lipoic acid in microencapsulated or coated form (protected form for granules for sachets) or coated using a polymeric matrix (protected form for tablets) in the form of a non-dissociated acid or a salt, preferably a sodium salt.

The formulation of the present invention, both in the form of tablets and of granules per sachets, must have a controlled and gradual release over time to ensure a level of lipoic acid in blood serum from 0 to 4 hours. The formulation has a concentration comprised from 100 to 1200 milligrams/daily dose, even more preferably from 300 to 800 milligrams/daily dose.

The formulation comprises or, alternatively, consists of an association between:
(i) folic acid or vitamin B or folacin,
(ii) myo-inositol, and
(iii) lipoic acid in the form of a 50/50 racemic R-(+)-alpha lipoic acid/S-(-)-alpha lipoic acid mixture, said lipoic or alpha lipoic acid being in microencapsulated form or coated with a matrix (protected form), for use in the treatment of infertility.

In many cases, female infertility occurs as a result of chronic anovulation. The alterations of the menstrual cycle comprise, for example, oligomenorrhoea and amenorrhoea. Alterations of the menstrual cycle may lead to very severe pains.

In one embodiment, the formulation of the present invention, in the form of granules for sachets or hard capsules, consists of vitamin B9 (or folic acid or folacin), myo-inositol and R-(+)-alpha lipoic acid or a 50/50 racemic mixture, the latter in a microencapsulated or coated form (alpha lipoic acid protected for granules or for tablets) with a controlled and gradual release over a period of from 0 to 4 hours.

The formulation of the present invention (pharmaceutical composition or nutraceutical composition or supplement product or food supplement) comprises a qualitative/quantitative chemical composition having the characteristics as set forth above, in combination with one or more excipients or additives, as described above. The formulation is prepared using apparatus and methods known to a person skilled in the art, who is undoubtedly capable of selecting the best operating conditions according to the type of formulation to be realized.

Initially, one proceeds to prepare a homogeneous and finely subdivided mixture. Said mixture will comprise vitamin B9 or folic acid or folacin -(i), myo-inositol - (ii), alpha lipoic acid -(iii) and the excipients and/or technological additives and/or co-formulants and/or polar and/or semipolar polymeric matrices and/or carriers and/or stabilizers according to the form of administration it is desired to prepare.

Said mixture is prepared by adding the various components in sequence inside a container equipped with stirring means and heating means.

It is also possible to use several types of matrices selected from among lipid matrices, carboxymethyl cellulose, carboxypropyl cellulose, hydroxypropyl cellulose and lacquer gum in order to formulate the composition in the desired form of administration.

Subsequently, using known apparatus and methods well known in the pharmaceutical and dietary supplements industry, a formulation is prepared in solid, granular or powder form, which is suitable for oral administration in the form of pills, tablets, softgels or orosoluble pharmaceutical forms.

The pharmaceutical composition or supplement product of the present invention shows a surprisingly positive effect in the treated subjects.

Even though at present the mechanism of action has not been completely clarified, it seems that the effectiveness of the present composition is due to a synergistic effect among vitamin B9 or folic acid or folacin, myo-inositol and alpha lipoic acid contained in said composition. Furthermore, an additional effect has emerged, ascribable to a specific dose/regime of administration.

The pharmaceutical composition or the supplement product of the present invention can be administered once or twice a day for the purpose of administering to the subject undergoing treatment a daily dose as described above for each individual active component.

The pharmaceutical composition or supplement product of the present invention can be administered both as a background therapy and a periodic therapy.

In a preferred embodiment, a sachet or a tablet is administered (permanent background therapy).

As example of a granular formulation in sachets (2.5 g) comprises:
- Folic acid or vitamin B or folacin: 0.10 mg;
- Inositol, myo-inositol: 1000 mg;
- Coated lipoic acid, alpha lipoic acid: 400mg.
- Additives: approx. 1100 mg:
   o Stabilizers: sorbitol, guar gum;
   o Thickening agents: xanthan gum, maltodextrins;
   o Acidifier: citric acid;
   o Anti-agglomerant: silicon dioxide;
   o Edulcorant: sucralose.

In a preferred embodiment, two sachets or two tablets are administered per day for a period comprised from 4 to 20 weeks.

The formulation of the present invention was tested on non-diabetic patients suffering from polycystic ovary syndrome (PCOS) in order to evaluate the effects of administering said formulation on several specific parameters of the polycystic ovary syndrome.
Group 1: 10 female patients with age ranging from 20 to 40 years took a formulation based on metformin, according to the recommended dose, on an empty stomach and between meals, for 3 months.
Group 2: 10 female patients with age ranging from 20 to 40 years took a formulation based on inositol and antioxidants, 1200 mg/day (Redestop^{®} - Progine), on an empty stomach and between meals, for 3 months.
Group 3: 10 female patients with age ranging from 20 to 40 years took a formulation based on coated alpha lipoic acid in granules, 1200 mg/day (Tiobec 600^{®} - Laborest Italia Spa), on an empty stomach and between meals, for 3 months.
Group 4: 10 female patients with age ranging from 20 to 40 years took a formulation of the present invention comprising (per 5000 mg/day of total formulation) 0.2 mg folic acid, 2000 mg myo-inositol and 800 mg coated alpha lipoic acid, on an empty stomach and between meals, for 3 months.

The treated subjects belonging to group 4 showed significant improvements compared to the subjects in groups 2 and 3 with respect to the parameters below (i)-(vii).

The following parameters were evaluated and compared among the different groups 1-4:
i) insulin sensitivity, based on insulinaemia,
ii) glucose tolerance, based on the Homa and Quicki indexes,
iii) ovulation, based on progesterone levels,
iv) the serum concentration of androgens, via assay of ovarian, suprarenal and peripheral metabolic androgens,
v) total cholesterol levels, via plasma assay,
vi) LDL cholesterol levels, via plasma assay,
vii) HDL cholesterol levels, via plasma assay.

A lowering of triglyceride values, increase in insulin sensitivity and a distinct improvement in ovulation were observed. Furthermore, a decrease in insulin circulation and in total serum testosterone was observed, along with an improvement in metabolic factors.

## Claims

1. A composition comprising:
- vitamin B9 or folic acid or folacin, and
- myo-inositol, and
- alpha lipoic acid,
for use in the treatment of infertility.

2. The composition for use in accordance with claim 1, wherein said alpha lipoic acid is present as a 50/50 racemic R-(+)-alpha lipoic acid/S-(-)-alpha lipoic acid mixture.

3. The composition for use in accordance with claim 1 or 2, wherein said acid alpha lipoic is present in microencapsulated or coated form.

4. The composition for use in accordance with any one of the preceding claims, wherein said composition consists of:
- vitamin B9 or folic acid or folacin, preferably pteroylmonoglutamic acid;
- myo-inositol;
- alpha lipoic acid in the form of a 50/50 racemic R-(+)-alpha lipoic acid/S-(-)-alpha lipoic acid mixture, and excipients and technological additives.

5. The composition for use in accordance with any one of the preceding claims, wherein said composition is in solid form for oral administration, preferably tablets or granules for sachets or hard capsules.

6. The composition for use in accordance with any one of the preceding claims as a pharmaceutical composition or supplement product.

## Patentansprüche

1. Zusammensetzung, umfassend:
- Vitamin B9 oder Folsäure oder Folacin, und
- myo-Inosit, und
- Alpha-Liponsäure,
zur Verwendung bei der Behandlung von Unfruchtbarkeit.

2. Zusammensetzung zur Verwendung gemäß Anspruch 1, worin die Liponsäure als 50/50 racemische R-(+)-alpha-Liponsäure/S-(-)-alpha Liponsäure-Mischung vorliegt.

3. Zusammensetzung zur Verwendung gemäß Anspruch 1 oder 2, worin die Alpha-Liponsäure in mikroverkapselter oder beschichteter Form vorliegt.

4. Zusammensetzung zur Verwendung gemäß einem der vorhergehenden Ansprüche, wobei die Zusammensetzung besteht aus:
- Vitamin B9 oder Folsäure öder Folacin, vorzugsweise Pteroylmonoglutaminsäure;
- myo-Inosit;
- Alpha-Liponsäure in Form einer 50/50 racemischen R-(+)-alpha-Liponsäure/S-(-)-alpha Liponsäure-Mischung, und Hilfsstoffen und technologischen Zusatzstoffen.

5. Zusammensetzung zur Verwendung gemäß einem der vorhergehenden Ansprüche, worin die Zusammensetzung in fester Form zu oralen Verabreichung, vorzugsweise in Form von Tabletten oder Granalien für Sachets oder Hartkapseln, vorliegt.

6. Zusammensetzung zur Verwendung gemäß einem der vorhergehenden Ansprüche als pharmazeutische Zusammensetzung oder Ergänzungsmittel.

## Revendications

1. Composition comprenant :
- de la vitamine B9 ou de l'acide folique ou de la folacine, et
- du myo-inositol, et
- de l'acide alpha lipoïque,
pour une utilisation dans le traitement de l'infertilité.

2. Composition pour une utilisation selon la revendication 1, dans laquelle ledit acide alpha lipoïque est présent sous la forme d'un mélange racémique 50/50 d'acide R-(+)-alpha lipoïque/acide S-(-)-alpha lipoïque.

3. Composition pour une utilisation selon la revendication 1 ou 2, dans laquelle ledit acide alpha lipoïque est présent sous forme microcapsulée ou enrobée.

4. Composition pour une utilisation selon l'une quelconque des revendications précédentes, dans laquelle ladite composition est constituée de :
- vitamine B9 ou acide folique ou folacine, de préférence d'acide ptéroylmonoglutamique ;
- myo-inositol ;
- acide alpha lipoïque sous la forme d'un mélange racémique 50/50 d'acide R-(+)-alpha lipoïque/acide S-(-)-alpha lipoïque, et d'excipients et d'additifs technologiques.

5. Composition pour une utilisation selon l'une quelconque des revendications précédentes, dans laquelle ladite composition est sous forme solide pour une administration orale, de préférence des comprimés ou des granules pour sachets ou des gélules dures.

6. Composition pour une utilisation selon l'une quelconque des revendications précédentes en tant que composition pharmaceutique ou produit de supplémentation.
